# EUROPEAN PATENT APPLICATION

(11) **EP 4 527 418 A1**
(43) Date of publication of application: **26.03.2025**
(21) Application number: 23826936.9
(22) Date of filing: 31.05.2023
(51) Int. Cl.: A61L 2/10

(54) **FLUID STERILIZATION DEVICE**

(30) Priority: 21.06.2022 JP 2022099469
(71) Applicant: Stanley Electric Co. Ltd., Tokyo 153-8636 (JP)
(72) Inventor: TANAKA Hideaki, Tokyo 153-8636 (JP); KATO Hiroyuki, Tokyo 153-8636 (JP); SHINNO Kazuhisa, Tokyo 153-8636 (JP)
(74) Representative: Schmidbauer, Andreas Konrad
(86) International application number: PCT/JP2023/020332
(87) International publication number: WO 2023/248749

(57) **Abstract**

Straight tubes 16a, 16b respectively include fluid paths Pa, Pb, and are joined with a circuit substrate 40 interposed therebetween in the lateral direction. LEDs 44a, 44b are respectively mounted on the fluid paths Pa, Pb sides of the circuit substrate 40, and fluids in the fluid paths Pa, Pb are respectively irradiated with ultraviolet light beams La, Lb. The heat generated by the LEDs 44a, 44b is diffused from heat sinks 46a, 46b at a rear surface position to the fluids of the fluid paths Pb, Pa.

## Description

### Technical Field

The present invention relates to a fluid sterilization device that sterilizes a fluid flowing in a fluid path with ultraviolet light beams.

### Background Art

A fluid sterilization device that irradiates a fluid flowing in a fluid path with ultraviolet light beams to sterilize bacteria contained in the fluid is known.

Patent Document 1 discloses a fluid sterilization device including a cylindrical member, a plurality of LEDs that are mounted on a peripheral portion of the cylindrical member at regular intervals in an axial direction and a circumferential direction and radiate ultraviolet light beams, and a cylindrical tube that is coaxially disposed on the cylindrical member to surround the cylindrical member and forms a fluid path through which water to be sterilized flows in the axial direction on an inner peripheral side. In this fluid sterilization device, heat generated by the LEDs is thermally conducted to the cylindrical portion and is released to flowing water from the surface of the peripheral portion of the cylindrical portion, so that the LED can be efficiently cooled.

### Citation List

### Patent Literature

Patent Literature 1: Japanese Patent No. 6180178

### Summary of Invention

### Technical Problem

In the fluid sterilization device of Patent Document 1, since the water to be sterilized flows over the entire circumference of the cylindrical member, the number of LEDs in the circumferential direction increases and the peripheral length of the cylindrical member also increases in order to irradiate the entire circumference with ultraviolet light beams thoroughly. This hinders the miniaturization of the fluid sterilization device.

An object of the present invention is to provide a fluid sterilization device that can achieve the miniaturization while efficiently diffusing heat generated by a light source of ultraviolet light beams to a fluid to be sterilized.

### Solution to Problem

According to the present invention, there is provided a fluid sterilization device including a first tube and a second tube that have a first fluid path and a second fluid path that extend in a first direction in parallel to each other, and configured to be joined to each other; a circuit substrate configured to be interposed between the first tube and the second tube; a first group of light sources configured to be disposed along the first fluid path on a surface of a side of the first fluid path of the circuit substrate, and configured to irradiate the first fluid path with ultraviolet light beams; a first group of heat sinks configured to be disposed at a rear surface position of each of the light sources in the first group on a surface of a side of the second fluid path of the circuit substrate; a second group of light sources configured to be disposed at positions which do not overlap the first group of heat sinks in the first direction on the surface of the side of the second fluid path of the circuit substrate, and configured to irradiate the second fluid path with ultraviolet light beams; and a second group of heat sinks configured to be disposed at a rear surface position of each of the light sources in the second group on the surface of the side of the second fluid path of the circuit substrate on the surface of the side of the first fluid path of the circuit substrate.

### Effect of the Invention

According to the present invention, the first fluid path and the second fluid path are separately formed on both sides of the circuit substrate, and the first group and the second group of light sources are disposed on the surfaces of the side of the first fluid path and the side the second fluid path of the circuit substrate, respectively, and irradiate the first fluid path and the second fluid path with ultraviolet light beams to perform sterilization. In addition, the first group and the second group of the heat sinks are respectively disposed at the rear surface positions of the light sources of the first group and the second group on the circuit substrate, and diffuse heat to the fluids of the second fluid path and the first fluid path. Accordingly, it is possible to achieve the miniaturization by devising the disposition of the light sources while having a structure in which heat generated by the light sources is effectively diffused to the fluid.

### Brief Description of Drawings

FIG. 1 is an exploded view of a fluid sterilization device according to a first embodiment, which is exploded in a longitudinal direction.
FIG. 2 is an exploded view of a coupling portion of a straight tube in a lateral direction.
FIG. 3 is a longitudinal cross-sectional view taken along a direction in which two straight tubes of the fluid sterilization device of the first embodiment are arranged.
FIG. 4 is a cross-sectional view taken along a line A4-A4 of FIG. 3.
FIG. 5A is a view illustrating an irradiation region of ultraviolet light beams L in the fluid sterilization device according to the first embodiment.
FIG. 5B is a diagram illustrating an irradiation region of ultraviolet light beams in a case where all the LEDs irradiate a first tube side with ultraviolet light beams La.
FIG. 5C is a diagram illustrating an irradiation region of ultraviolet light beams in a case where all the LEDs irradiate a second tube side with the ultraviolet light beams La.
FIG. 6 is a longitudinal cross-sectional view of a fluid sterilization device according to a second embodiment.
FIG. 7 is a longitudinal cross-sectional view of a fluid sterilization device according to a third embodiment.
FIG. 8 is a longitudinal cross-sectional view of a fluid sterilization device according to a fourth embodiment.

### Description of Embodiments

Hereinafter, preferred embodiments of the present invention will be described, but these embodiments may be appropriately modified and combined. In addition, in the following descriptions and the accompanying drawings, substantially the same or equivalent parts will be described with the same reference numerals.

### (First Embodiment)

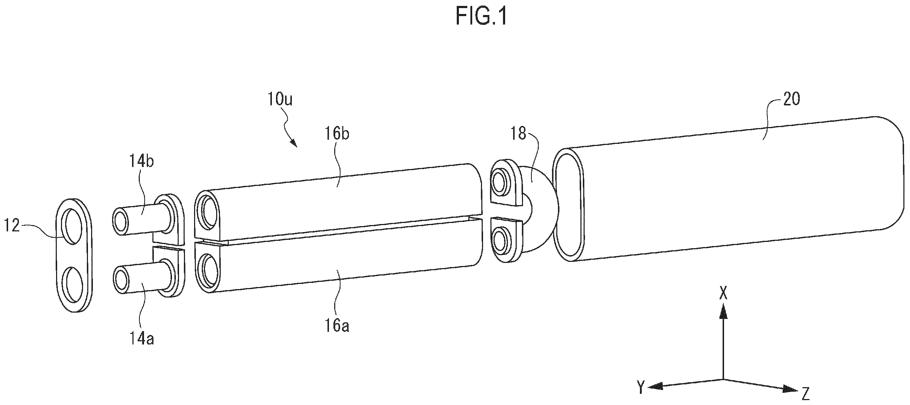

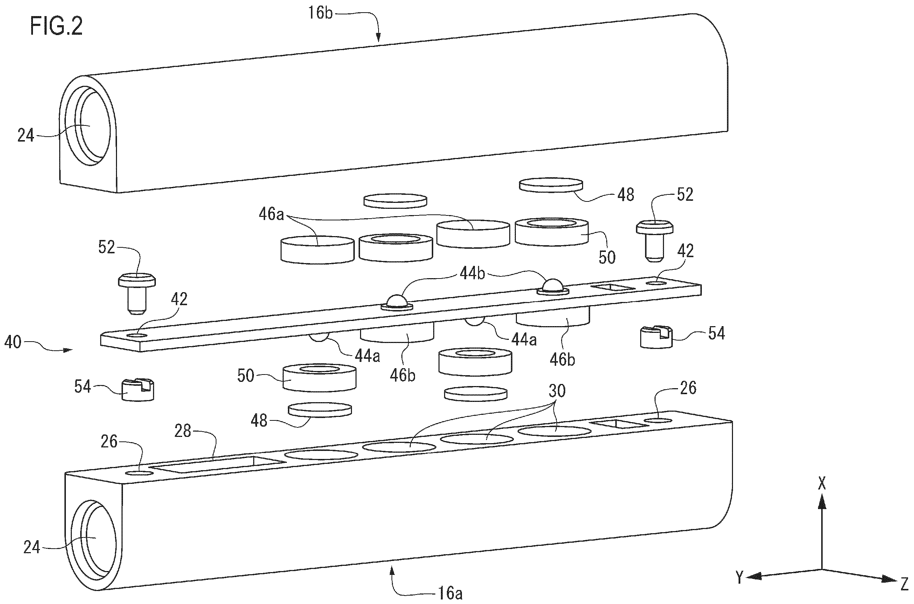

FIG. 1 is an exploded view of a fluid sterilization device 10u according to a first embodiment, which is exploded in a longitudinal direction. In the exploded view, the components interposed between a straight tube 16a and a straight tube 16b are not shown. These components will be described later with reference to FIG. 2.

The fluid sterilization device 10u includes a pressing plate 12, a pair of external connection tubes 14a and 14b, a pair of straight tubes 16a and 16b, a U-shaped tube 18, and a resin case 20 from one end side (+ side in the Y axis direction) to the other end side (- side in the Y axis direction) in a longitudinal direction (the Y axis direction as an example of the first direction). The pair of the external connection tubes 14a and 14b and the pair of the straight tubes 16a and 16b are arranged in the lateral direction (X axis direction) which is perpendicular to the longitudinal direction.

The resin case 20 has one end open and the other end closed in the Y axis direction. The fluid sterilization device 10u is assembled by sequentially inserting the U-shaped tube 18, the pair of the straight tubes 16a and 16b, and the pair of the external connection tubes 14a and 14b into the resin case 20 from the opening side, and then blocking the opening of the resin case 20 by the pressing plate 12. Since the pressing plate 12 presses the flange portions of the external connection tubes 14a and 14b against the opening end portion of the resin case 20, the flange portions of the external connection tubes 14a and 14b are hidden in the resin case 20. On the other hand, the tube portions of the external connection tubes 14a and 14b penetrate the respective through-holes of the pressing plate 12 and are exposed to the outside of the resin case 20.

FIG. 2 is an exploded view of the coupling portion of the straight tubes 16a and 16b in the lateral direction. The straight tubes 16a and 16b have the same structure and dimensions, and only the orientations in the lateral direction are opposite to each other in the fluid sterilization device 10u. Hereinafter, in the fluid sterilization device 10u, in a case where each pair of individual elements is collectively referred to without distinction, the elements are indicated by reference numerals with a, b at the end of the reference numerals omitted.

The straight tubes 16a and 16b are inserted into the resin case 20 while the rectangular planes are aligned with each other with a circuit substrate 40 interposed therebetween, with the joining side (ventral side) being a rectangular plane, and are mutually joined by the resin case 20. In the rectangular circuit substrate 40, the dimension in the longitudinal direction (Y axis direction) is the same and the dimension in the width direction (Z axis direction) is the same or slightly smaller than that of the rectangular plane of the straight tube 16. In the rectangular plane of the straight tube 16, the screw insertion hole 26, the circuit element housing hole 28, the plurality of columnar recess portions 30, and the screw insertion hole 26 are provided in order from one end side to the other end side in the Y axis direction in a row.

In the circuit substrate 40, a through-hole 42 is formed at the same position as each screw insertion hole 26 of the straight tube 16 in the Y axis direction. A female screw 54 is press-fitted into the screw insertion hole 26 of the straight tube 16a and fixed thereto prior to the fixing of the circuit substrate 40 to the straight tube 16a. A male screw 52 is inserted into the through-hole 42 of the circuit substrate 40 from the side of the straight tube 16b, and the shaft portion is screwed to the female screw 54. Accordingly, the circuit substrate 40 is fixed to the straight tube 16a. After the head portions of the male screws 52 are joined to the straight tubes 16a and 16b and then stored in the screw insertion holes 26 of the straight tube 16b.

Unlike the example of FIG. 2, the circuit substrate 40 can also be fixed to the straight tube 16b. In that case, the female screw 54 is fixed in the screw insertion hole 26 of the straight tube 16b, and the male screw 52 is inserted into the through-hole 42 from the side of the straight tube 16a and then is screwed to the female screw 54 of the straight tube 16b.

The LED 44a and the heat sink 46b are mounted at the same positions as the recess portions 30 of the straight tube 16a corresponding in the Y axis direction on the surface of the circuit substrate 40 on the side of the straight tube 16a in an alternating arrangement in the Y axis direction. The LED 44b and the heat sink 46a are mounted at the same positions as the recess portion 30 of the straight tube 16b corresponding in the Y axis direction on the surface of the circuit substrate 40 on the side of the straight tube 16b in an alternating arrangement in the Y axis direction. Since the recess portion 30 of the straight tube 16a and the recess portion 30 of the straight tube 16b are formed at the same position in the Y axis direction, in the circuit substrate 40, the heat sinks 46a and 46b are positioned at the rear surface positions of the LEDs 44a and 44b, respectively.

Each of the ultraviolet transmission window 48 (for example, made of quartz glass) and a tubular body 50 is inserted into the recess portion 30 in a positional relationship with the bottom surface side and the opening side of the recess portion 30. The inner peripheral surface of the tubular body 50 is formed on a side surface of a truncated cone of which the diameter expands in a light emission direction from the LED 44. The tubular body 50 surrounds the LED 44 in a state of being inserted into the recess portion 30, and the ultraviolet transmission window 48 is exposed to the through-hole 31 on the bottom surface side of the recess portion 30 and blocks the recess portion 30 from the side of the through-hole 31. The tapered inner peripheral surface of the tubular body 50 is a mirror.

In FIG. 2, the ultraviolet transmission window 48 has a flat plate shape, but may have a lens shape for realizing a desired light distribution.

Although not shown in FIG. 2, the circuit substrate 40 has a circuit element (not shown) such as a driving element of the LED 44, in addition to the LED 44 and the heat sink 46. These circuit elements fit into the circuit element housing hole 28 in an assembled state of the fluid sterilization device 10u.

Examples of the material of each component are as follows.
Pressing plate 12: ABS resin or polypropylene
External connection tubes 14a and 14b: resin or fluororesin
Straight tubes 16a and 16b: fluororesin or metal (for example, stainless steel)
U-shaped tube 18: resin or fluororesin
Resin case 20: ABS resin or polypropylene
Heat sinks 46a and 46b: metal (for example, stainless steel) or ceramic
Tubular body 50: fluororesin
Male screw 52: stainless steel
Female screw 54: brass

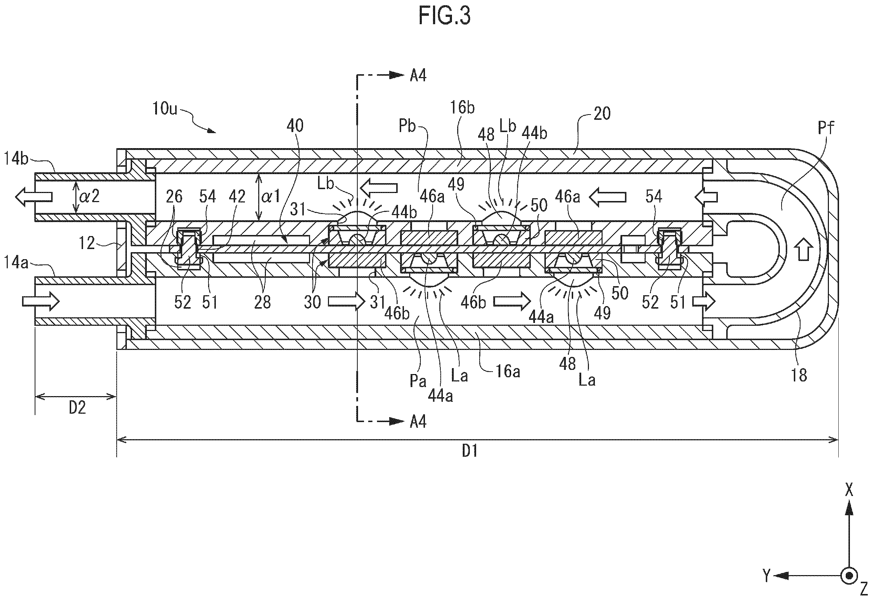

FIG. 3 is a longitudinal cross-sectional view of the fluid sterilization device 10u taken along a direction (X axis direction) in which two straight tubes 16 are arranged. The orientation of the fluid sterilization device 10u of FIG. 3 is a horizontally placed orientation in which the Y axis direction is horizontal, but a typical fluid sterilization device 10u is disposed in a vertically placed orientation in which the external connection tube 14 and the U-shaped tube 18 are respectively above and below in the vertical direction.

In FIG. 3, a white arrow indicates a direction in which the fluid to be sterilized flows in the fluid sterilization device 10u. The fluid is not limited to a liquid such as water, and may be a gas such as air. In the fluid sterilization device 10u, the external connection tubes 14a and 14b are connected to external devices (for example, a pump, a liquid source, and a storage tank) to be respectively inlets and outlets. On the contrary, that is, the external connection tubes 14a and 14b can be connected to external devices to be respectively outlets and inlets.

The straight tubes 16a and 16b have fluid paths Pa and Pb therein. The U-shaped tube 18 has a fluid path Pf therein. The fluid to be sterilized flows into the fluid sterilization device 10u from the external connection tube 14a, and then flows in the order of the fluid path Pa → the fluid path Pf → the fluid path Pb, and flows out of the fluid sterilization device 10u from the external connection tube 14b.

The recess portion 30 is formed in a thick-walled portion of the tube wall of the straight tube 16, is open in a rectangular plane of the straight tube 16, and is a circular hole having a predetermined depth. The through-hole 31 extends in the radial direction of the straight tube 16 between the bottom surface of the recess portion 30 and the inner peripheral surface of the straight tube 16 to communicate the both with each other. The diameter and the depth of the recess portion 30 are substantially equal to the diameter and the thickness of the heat sink 46. The diameter of the through-hole 31 is slightly smaller than the diameter of the recess portion 30, and is a dimension that prevents the heat sink 46, the ultraviolet transmission window 48, and the tubular body 50 in the recess portion 30 from being separated from the recess portion 30 to the inner peripheral side of the straight tube 16.

The recess portions 30 are arranged in a row in the axial direction in the straight tube 16. The LED 44a and the heat sink 46b are alternately stored in the recess portion 30 of the straight tube 16a in a row direction. The LED 44a and the heat sink 46a having a rear surface position relationship with each other are mounted on the surfaces of the circuit substrate 40 on the side of the fluid path Pa and the side of the fluid path Pb at the same position in the axial direction. The LED 44b and the heat sink 46b having a rear surface position relationship with each other are mounted on the surfaces of the circuit substrate 40 on the side of the fluid path Pb and the side of the fluid path Pa, respectively, at the same position in the axial direction.

The annular tubular body 50 is fitted into the recess portion 30 in which the LED 44 is disposed, and surrounds the LED 44. The ultraviolet transmission window 48 is inserted into the through-hole 31 from the recess portion 30 side to block the side of the fluid path Pa or Pb of the tubular body 50. The O-ring 49 closely holds the peripheral portion of the ultraviolet transmission window 48 and the peripheral wall of the recess portion 30. The ultraviolet light beams L radiated from the LED 44 pass through the ultraviolet transmission window 48 and are applied to the fluid of the fluid path Pa or the fluid path Pb.

In FIG. 3, D1 indicates a dimension of a total of the length of the pressing plate 12 and the length of the resin case 20 in the Y axis direction. D2 indicates a protruding dimension of the external connection tube 14 from the pressing plate 12 in the Y axis direction. D1 is, for example, 150 mm. D2 is, for example, 17 mm.

In FIG. 3, α1 and α2 indicate diameters of the straight tube 16 and the external connection tube 14, respectively. α1 is, for example, ϕ10 mm. α2 is, for example, ϕ7 mm.

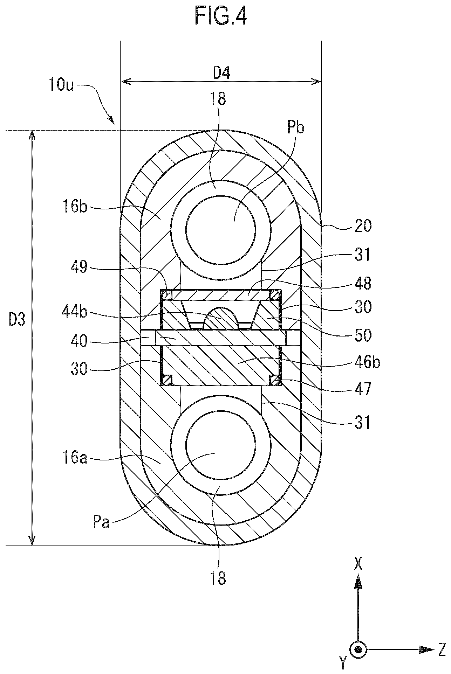

FIG. 4 is a cross-sectional view taken along the line A4-A4 of FIG. 3. As can be seen from FIG. 4, the straight tubes 16a and 16b are inserted into the resin case 20 with the circuit substrate 40 interposed therebetween in the X axis direction, and thus, all of the straight tubes 16a and 16b and the circuit substrate 40 are held in a pinched state in the X axis direction by the inner periphery of the resin case 20. Therefore, since the circuit substrate 40 can be smoothly fixed to the straight tube 16 even without fixing the circuit substrate 40 to the straight tube 16 with the male screw 52, the male screw 52 and the female screw 54 can be omitted.

The O-ring 47 is fitted between the peripheral wall of the recess portion 30 and the peripheral portion of the heat sink 46, and holds liquid-tightness therebetween.

The peripheral portion of the heat sink 46 is in contact with the peripheral surface of the recess portion 30 of the straight tube 16. Therefore, the heat generated by the light emission of the LED 44 is diffused to the fluid of the fluid path Pa or the fluid path Pb by the first heat transfer path which reaches the fluid path Pa or the fluid path Pb from the bottom surface of the heat sink 46 via the fluid flowing into the through-hole 31, and is additionally conducted from the peripheral portion of the heat sink 46 to the straight tube 16, is conducted to the straight tube 16 in the circumferential direction, and, further diffused to the fluid of the fluid path Pa or the fluid path Pb in the second heat transfer path through which reaches the fluid path Pa or the fluid path Pb from the inner peripheral surface of the straight tube 16.

In addition, since the circuit substrate 40 is in contact with the rectangular plane of the straight tube 16, heat generated by the operation of the LED 44 and additionally mounted driving circuit is conducted to the straight tube 16 and is conducted to the second heat transfer path.

The action of the fluid sterilization device 10u will be described. A fluid to be sterilized (for example, water) is pumped from a pump (not shown) and flows into the fluid sterilization device 10u from the external connection tube 14a. As shown by a white arrow in FIG. 3, the fluid flows (proceeds) from one end side to the other end side of the fluid path Pa in the axial direction, then makes a U-turn in the fluid path Pf, then flows from the other end side to one end side of the fluid path Pb in the axial direction, and flows out of the fluid sterilization device 10u from the external connection tube 14b.

Each LED 44 of the straight tube 16 irradiates the fluid of the fluid path Pa or the fluid path Pb with the ultraviolet light beams to sterilize the bacteria in the fluid in the fluid. The LED 44 generates heat with light emission. As described above, the heat is conducted in both the first heat transfer path and the second heat transfer path and is diffused to the fluids in the fluid paths Pa and Pb. Although the amount of heat transfer in the second heat transfer path is smaller than the amount of heat transfer in the first heat transfer path, the heat can be diffused to the fluid flowing through the fluid paths Pa and Pb from the entire inner periphery of the tube wall of the straight tube 16, and thus the heat diffusion area increases.

### (Comparison)

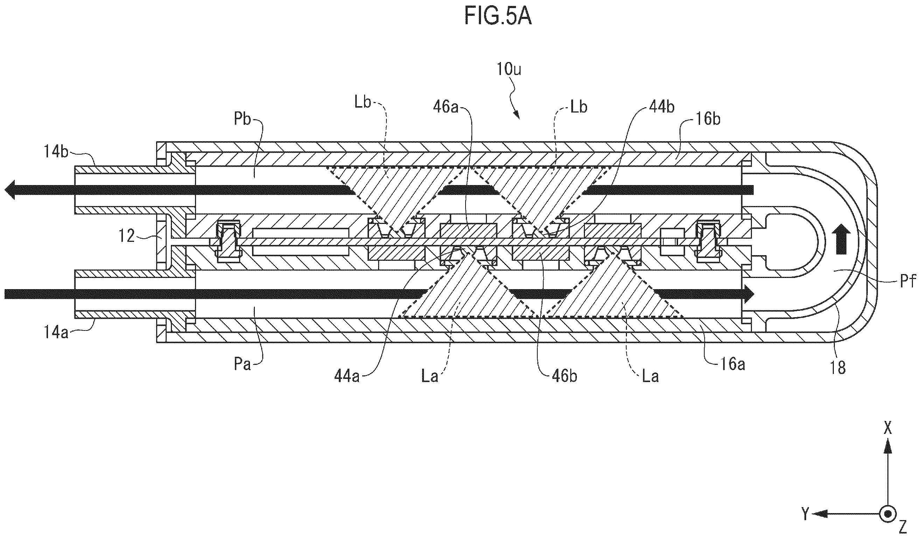

FIGS. 5A to 5C are explanatory diagrams of the significance of alternately arranging the LED 44 and the heat sink 46 on both surfaces of the circuit substrate 40. In FIGS. 5A to 5C, a region of an isosceles triangle hatched with diagonal lines indicates an irradiation region of the ultraviolet light beam La or Lb.

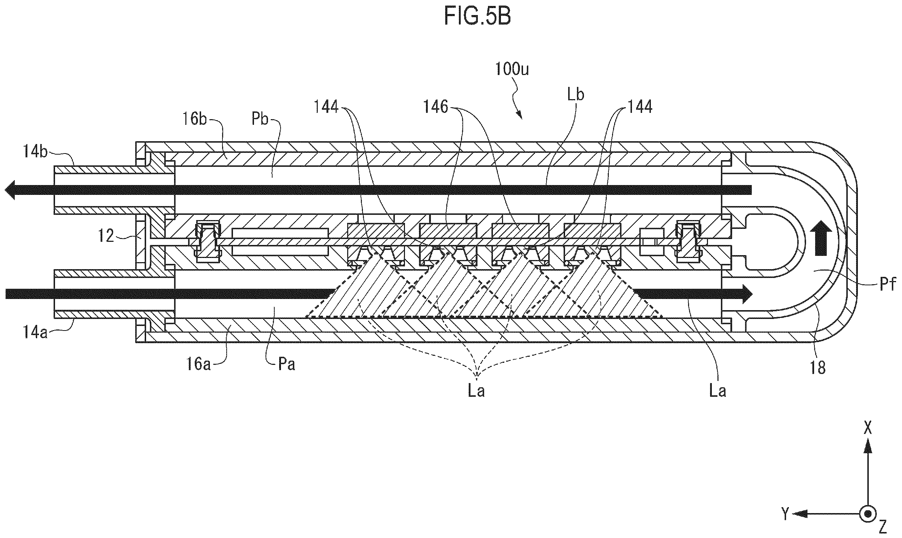

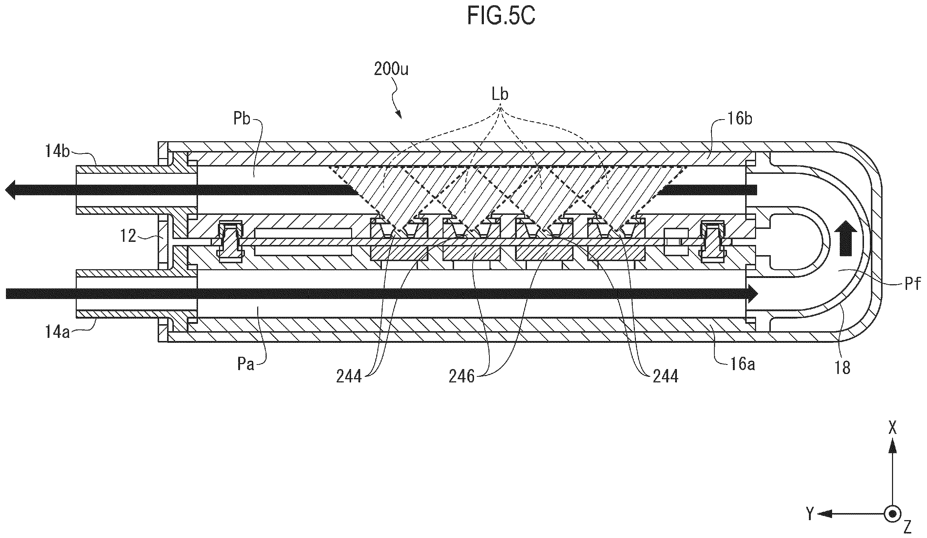

In the fluid sterilization device 10u of FIG. 5A, the LED 44 and the heat sink 46 are alternately arranged on both surfaces of the circuit substrate 40. On the other hand, in the fluid sterilization device 100u of FIG. 5B and the fluid sterilization device 200u of FIG. 5C, all the LEDs 44 or all the heat sinks 46 are arranged on only one surface of the circuit substrate 40. The total number of LEDs is the same in the fluid sterilization devices 10u, 100u, and 200u. In addition, the interval between the LEDs 44 in the Y axis direction in both surfaces of the circuit substrate 40 is also made equal for all the fluid sterilization devices 10u, 100u, and 200u, and the comparison between the fluid sterilization devices 10u, 100u, and 200u is performed.

FIG. 5A shows an irradiation region of ultraviolet light beams in the fluid sterilization device 10u. The ultraviolet light beams La and Lb are ultraviolet light beams from the heat sinks 46a and 46b, respectively. Since the heat sinks 46a and 46b respectively irradiate the fluid paths Pa and Pb with the ultraviolet light beams La and Lb in the circuit substrate 40, the ultraviolet light beams from the adjacent LEDs 44 in the Y axis direction does not overlap, and the fluid is irradiated with the ultraviolet light beams. That is, in the fluid sterilization device 10u, it is possible to secure a fluid path region to be irradiated with ultraviolet light beams having an intensity equal to or higher than a predetermined value.

FIG. 5B shows an irradiation region of ultraviolet light beams in a case where all the LEDs 144 are directed toward the side of the straight tube 16a and radiate the ultraviolet light beam La, and FIG. 5C shows an irradiation region of ultraviolet light beams in a case where all the LEDs 244 are directed toward the side of the straight tube 16b and radiate the ultraviolet light beam Lb. In FIG. 5B, since all the heat sinks 146 are mounted only on the side of the straight tube 16b of the circuit substrate 40, the heat of the heat sink 146 is diffused to the fluid flowing through the fluid path Pb. In FIG. 5C, since all the heat sinks 246 are mounted only on the side of the straight tube 16a of the circuit substrate 40, the heat of the heat sink 246 is diffused to the fluid of the straight tube 16a.

In the aspects of FIGS. 5B and 5C, since the fluid sterilization device is miniaturized in the same manner as the fluid sterilization device 10u and the mounting direction of the light source is unified, the production is facilitated.

### (Second Embodiment)

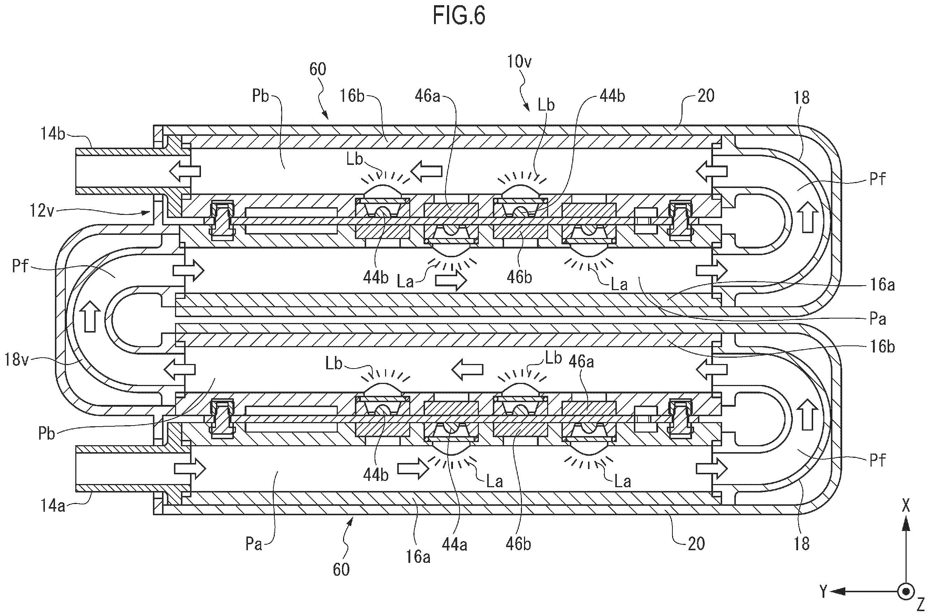

FIG. 6 is a longitudinal cross-sectional view of a fluid sterilization device 10v of a second embodiment. The longitudinal cross section of FIG. 6 is a cross section taken in the direction in which the straight tubes 16a and 16b are arranged. The sterilization pipeline unit 60 refers to a structure in which the external connection tubes 14a and 14b are removed from the fluid sterilization device 10u of FIG. 3. The fluid sterilization device 10v has a structure in which two sterilization pipeline units 60 are arranged in the X axis direction and are connected in series by a U-shaped tube 18v.

Specifically, the external connection tube 14a is connected to the straight tube 16a of the sterilization pipeline unit 60 on the lower side (the - side in the X axis direction), and the external connection tube 14b is connected to the straight tube 16b on the upper side (the + side in the X axis direction). The U-shaped tube 18v connects the straight tube 16b of the sterilization pipeline unit 60 on the lower side and the straight tube 16a of the sterilization pipeline unit 60 on the upper side.

In the fluid sterilization device 10v, the fluid flows into the sterilization pipeline unit 60 on the lower side from the external connection tube 14a, flows into the U-shaped tube 18v through the fluid path Pa → the fluid path Pf → the fluid path Pb of the sterilization pipeline unit 60 on the lower side, flows into the sterilization pipeline unit 60 on the upper side through the fluid path Pf of the U-shaped tube 18v, and flows out from the external connection tube 14b through the fluid path Pa → the fluid path Pf → the fluid path Pb of the sterilization pipeline unit 60 on the upper side.

In the fluid sterilization device 10v, the total length of the fluid path is twice that of the fluid sterilization device 10u, and the total irradiation amount of ultraviolet light beams with respect to the fluid is twice that of the fluid sterilization device 10u. As a result, the sterilization power can be strengthened.

In the fluid sterilization device 10v, although two sterilization pipeline units 60 are connected, the fluid path length is increased by connecting three or more sterilization pipeline units 60, so that the sterilization power can be further increased.

### (Third Embodiment)

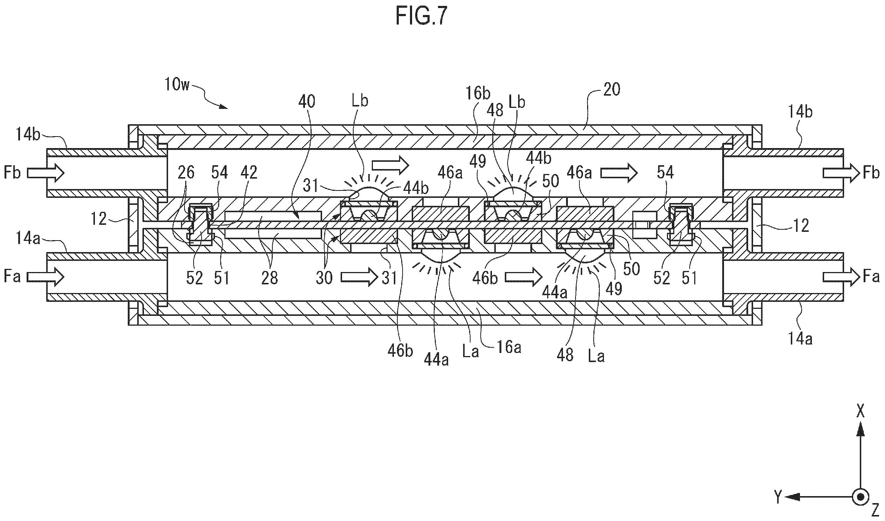

FIG. 7 is a longitudinal cross-sectional view of a fluid sterilization device 10w of a third embodiment. The longitudinal cross section of FIG. 7 is a cross section parallel to the arrangement direction of the straight tube 16a and the straight tube 16b. The differences between the fluid sterilization device 10w and the fluid sterilization device 10u of the first embodiment will be described. In the fluid sterilization device 10w, the U-shaped tube 18 of the fluid sterilization device 10u is omitted, and instead, external connection tubes 14a and 14b are connected to the other end sides of the straight tubes 16a and 16b in the Y axis direction, respectively.

The external connection tubes 14a and 14a connected to one end side and the other end side of the straight tube 16a as the first tube according to the present invention correspond to the first connection tube and the third connection tube according to the present invention, respectively. The external connection tubes 14b and 14b connected to one end side and the other end side of the straight tube 16b as the second tube of the present invention correspond to the second connection tube and the fourth connection tube of the present invention, respectively.

The fluids to be sterilized flowing through the straight tubes 16a and 16b are denoted by Fa and Fb, respectively. The fluids Fa and Fb linearly flow through the fluid path Pa and the fluid path Pb of the straight tube 16b from one end side to the other end side in the Y axis direction, respectively. The fluids Fa and Fb are irradiated with ultraviolet light beams from the LEDs 44a and 44b for a period of time during which the fluids Fa and Fb flow through the fluid paths Pa and Pb, respectively, and are sterilized.

The fluids Fa and Fb may be the same fluid or different fluids. In a case where the fluids Fa and Fb are the same fluid, a branch tube and a confluence tube can be connected to the upstream side and the downstream side of the fluid sterilization device 10w, respectively. As a result, the flow rate of the fluid to be sterilized can be doubled.

The temperatures of the fluids Fa and Fb can also be made different from each other. That is, the fluids Fa and Fb are, for example, hot water and cold water, respectively, and both the hot water and the cold water can be simultaneously sterilized using the fluid sterilization device 10w.

In the example of FIG. 7, the fluids Fa and Fb flow in the same direction in the Y axis direction, but can also flow in opposite directions to each other.

### (Fourth Embodiment)

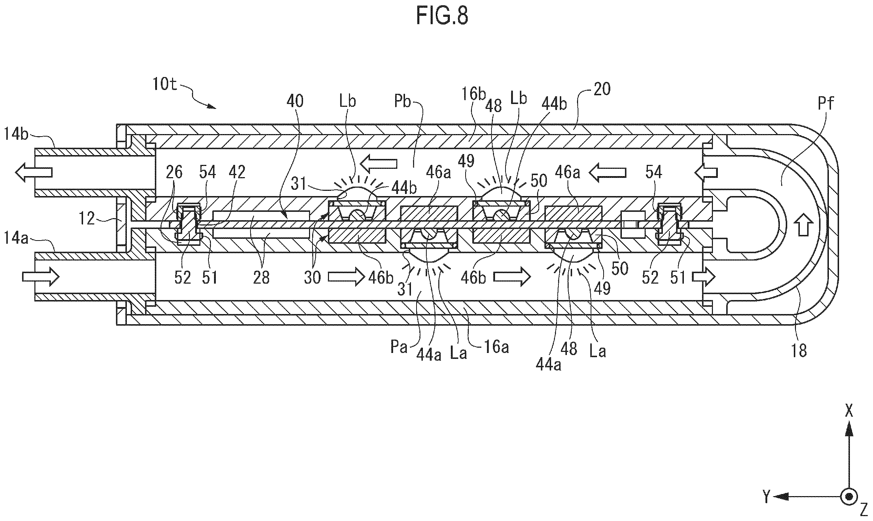

FIG. 8 is a longitudinal cross-sectional view of a fluid sterilization device 10t of a fourth embodiment. The longitudinal cross section of FIG. 8 is a cross section parallel to the arrangement direction of the straight tube 16a and the straight tube 16b. The differences between the fluid sterilization device 10t and the fluid sterilization device 10u of the first embodiment will be described. In the fluid sterilization device 10t, the through-hole 31 does not communicate with the recess portion 30 into which the heat sink 46 is inserted. Therefore, since the bottom surface of the heat sink 46 is not exposed to the fluid paths Pa and Pb, the heat of the heat sink 46 is not directly released to the fluids in the fluid paths Pa and Pb, and the heat of the heat sink 46 is diffused only by the second heat transfer path described above.

In the present embodiment, since the bottom surface of the heat sink 46 is not exposed to the fluid paths Pa and Pb, the heat sink 46 and the circuit substrate 40 and the mounted components are less likely to come into contact with the fluid, and reliability can be improved. In addition, the sealing member can be omitted and the cost can be reduced.

### (Modified Examples)

The straight tubes 16a and 16b correspond to a first tube and a second tube of the present invention, respectively. Similarly, the heat sinks 46a and 46b correspond to a first group of heat sinks and a second group of heat sinks of the present invention, respectively. The straight tubes 16a and 16b have the same shape and diameter, and the heat sinks 46a and 46b also have the same shape and diameter. However, the first tube and the second tube of the present invention may have different shapes and diameters, and the heat sinks 46a and 46b may also have different shapes and diameters. For example, the fluid path Pa of the straight tube 16a as a first fluid path may be thicker or thinner than the fluid path Pb of the straight tube 16b as a second fluid path.

The plurality of LEDs 44a constitute a first group of light sources, and the plurality of LEDs 44b constitute a second group of light sources. In the fluid sterilization device 10u and the like, the straight tube 16a and the straight tube 16b are alternately disposed in the Y axis direction on the circuit substrate 40, but in the present invention, the first group of light sources and the second group of light sources may be mounted on each surface of the circuit substrate in the longitudinal direction of the circuit substrate, and may not be alternately mounted. However, as described in FIGS. 5B and 5C, in a case where a plurality of light sources of the same group are continuously mounted on the circuit substrate, the irradiation efficiency of ultraviolet light beams may be reduced in a case where the interval is short. In addition, the number of light sources in the first group and the number of light sources in the second group may not be equal to each other.

The U-shaped tubes 18 and 18v correspond to a link tube of the present invention. The external connection tubes 14a and 14b correspond to a first connection tube and a second connection tube of the present invention, respectively.

The fluid sterilization device of the present invention is used for sterilizing, for example, water in a water storage tank of an ice maker or the like, water in a water supply tube, hot water in a water heater, water in a water server, cooling water in a circulation device (chiller), or water in a drink server.

### Description of Reference Numerals

10: fluid sterilization device
14a: external connection tube (first connection tube or third connection tube)
14b: external connection tube (second connection tube or fourth connection tube)
16a: straight tube (first tube)
16b: straight tube (second tube)
18, 18v: U-shaped tube (link tube)
30: recess portion
31: through-hole
40: circuit substrate
44a, 44b: LED (light source)
46a, 46b: heat sink
60: sterilization pipeline unit

## Claims

1. A fluid sterilization device comprising:
a first tube and a second tube that have a first fluid path and a second fluid path that extend in a first direction in parallel to each other, and configured to be joined to each other;
a circuit substrate configured to be interposed between the first tube and the second tube;
a first group of light sources configured to be disposed along the first fluid path on a surface of a side of the first fluid path of the circuit substrate, and configured to irradiate the first fluid path with ultraviolet light beams;
a first group of heat sinks configured to be disposed at a rear surface position of each of the light sources in the first group on a surface of a side of the second fluid path of the circuit substrate;
a second group of light sources configured to be disposed at positions which do not overlap the first group of heat sinks in the first direction on the surface of the side of the second fluid path of the circuit substrate, and configured to irradiate the second fluid path with ultraviolet light beams; and
a second group of heat sinks configured to be disposed at a rear surface position of each of the light sources in the second group on the surface of the side of the second fluid path of the circuit substrate on the surface of the side of the first fluid path of the circuit substrate.

2. The fluid sterilization device according to claim 1,
wherein the first group of light sources and the second group of light sources are alternately disposed in the first direction.

3. The fluid sterilization device according to claim 1,
wherein, in the first group of heat sinks and the second group of heat sinks, a surface of the side of the second fluid path and a surface of the side of the first fluid path are respectively exposed to the second fluid path and the first fluid path.

4. The fluid sterilization device according to claim 3,
wherein the first group of heat sinks and the second group of heat sinks are fitted into respectively formed recess portions, and the surface of the side of the second fluid path and the surface of the side of the first fluid path are respectively exposed to the second fluid path and the first fluid path via through-holes on bottom surfaces of the recess portions.

5. The fluid sterilization device according to claim 1, further comprising:
a first connection tube configured to be connected to one end portion of the first tube;
a second connection tube configured to be connected to one end portion of the second tube; and
a link tube configured to connect the other end portion of the first tube and the other end portion of the second tube to each other.

6. The fluid sterilization device according to claim 1, further comprising:
a first connection tube configured to be connected to one end portion of the first tube and a third connection tube configured to be connected to the other end portion of the first tube; and
a second connection tube configured to be connected to one end portion of the second tube and a fourth connection tube configured to be connected to the other end portion of the second tube.

7. A fluid sterilization device comprising:
a plurality of sterilization pipeline units; and
at least one link tube configured to connect the plurality of sterilization pipeline units in series, wherein
each of the sterilization pipeline units includes
a first tube and a second tube each have a first fluid path and a second fluid path that extend in a first direction in parallel to each other and communicate with each other at other end portions in the first direction, and configured to be joined to each other,
a circuit substrate configured to be interposed between the first tube and the second tube,
a first group of light sources configured to be disposed in a row along the first fluid path on a surface of a side of the first fluid path of the circuit substrate, and configured to irradiate the first fluid path with ultraviolet light beams,
a first group of heat sinks configured to be disposed at a rear surface position of each of the light sources in the first group on a surface of a side of the second fluid path of the circuit substrate,
a second group of light sources configured to be disposed in a row at positions which do not overlap with the first group of heat sinks on the surface of the side of the second fluid path of the circuit substrate in the first direction, and configured to irradiate the second fluid path with ultraviolet light beams, and
a second group of heat sinks configured to be disposed at a rear surface position of each of the light sources in the second group on the surface of the side of the second fluid path of the circuit substrate on the surface of the side of the first fluid path of the circuit substrate, and
the link tube links, with respect to two sterilization pipeline units among the plurality of sterilization pipeline units, the second tube of one sterilization pipeline unit and the first tube of the other sterilization pipeline unit on one end side in the first direction.
